# FASCICULE DE BREVET EUROPEEN

(11) **EP 1 470 836 B1**
(45) Date de publication et mention de la délivrance du brevet: **24.01.2007**
(21) Numéro de dépôt: 04290953.1
(22) Date de dépôt: 09.04.2004
(51) Int. Cl.: A61N 1/365, A61N 1/39

(54) **Dispositif médical implantable actif tel que stimulateur cardiaque, défibrillateur et/ou cardioverteur de type AAI ou AAI/DDD**
Implantierbares medizinisches aktives Gerät, wie Herzschrittmacher, defibrillator und/oder Kardioverter AAI oder AAI/DDD
Implantable active medical device like a cardiac stimulator, defibrillator and/or cardioverter AAI or AAI/DDD

(30) Priorité: 22.04.2003 FR 0304932
(43) Date de publication de la demande: 27.10.2004
(73) Titulaire: ELA MEDICAL, 92541 Montrouge (FR)
(72) Inventeur: Limousin, Marcel, 75014 Paris (FR); Amblard, Amel, 92290 Chatenay Malabry (FR)
(74) Mandataire: Dupuis-Latour, Dominique

(56) Documents cités:
- US-A1- 2001 029 392
- US-A1- 2002 095 183

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, dispositifs "multisite" (triple ou quadruple chambre), défibrillateurs et/ou cardioverteurs, permettant de délivrer au coeur des impulsions électriques de faible énergie pour le traitement des troubles du rythme cardiaque.

Elle concerne plus particulièrement ceux de ces dispositifs qui comprennent des circuits de stimulation et de détection à la fois sur l'oreillette et sur le ventricule et peuvent opérer selon deux modes de fonctionnement, DDD ou AAI (le mode AAI étant en fait un mode DDD comprenant un délai atrio-ventriculaire allongé). Ces dispositifs peuvent être pourvus d'un mode dénommé "DDD-CAM" assurant une commutation automatique du mode (CAM) de DDD en AAI et inversement.

Toutefois, comme on le verra par la suite, l'invention n'est pas limitée à ces dispositifs à commutation automatique de mode, mais peut s'appliquer à des dispositifs opérant en mode AAI seul, dans la mesure où, dans certaines configurations, l'invention propose seulement d'aménager le fonctionnement du mode AAI, sans qu'il y ait commutation automatique de mode et/ou pour éviter une commutation automatique de mode.

Le mode de fonctionnement de base d'un stimulateur DDD/AAI est un mode AAI, avec une stimulation auriculaire simple chambre et une surveillance (détection) de l'activité ventriculaire. Ce mode est maintenu tant que la conduction atrio-ventriculaire est normale, c'est-à-dire tant que chaque événement auriculaire (détection auriculaire, correspondant à une activité spontanée, ou stimulation auriculaire) est suivi d'une détection ventriculaire associée.

Dans certaines circonstances peuvent cependant apparaître des blocs atrio-ventriculaires (BAV) entraînant un défaut temporaire de dépolarisation du ventricule. Dans ce cas, le stimulateur bascule automatiquement en mode DDD automatique, avec des paramètres optimisés pour cette situation de BAV temporaire. Après disparition du BAV, et donc rétablissement de la conduction atrio-ventriculaire spontanée, dès lors qu'un certain nombre de conditions sont remplies le stimulateur retourne automatiquement au mode AAI.

Cette commutation des modes DDD et AAI est par exemple décrite dans le EP-A-0 488 904 (ELA Médical).

Le point de départ de l'invention réside dans un certain nombre d'observations effectuées lors d'un suivi clinique de patients appareillés avec des dispositifs AAI ou DDD/AAI à commutation automatique de mode, lorsque ces patients présentent des extrasystoles ventriculaires (ESV).

Les ESV sont des événements ventriculaires spontanés qui ne sont pas précédés d'une dépolarisation auriculaire et qui présentent un raccourcissement significatif de l'intervalle RR (ou VR) par rapport à l'événement ventriculaire précédent.

Selon le couplage de ces ESV par rapport à l'événement ventriculaire précédent, l'ESV peut survenir soit simultanément avec la dépolarisation auriculaire du battement suivant, soit à un instant proche de la fin de l'intervalle d'échappement auriculaire (IEA), c'est-à-dire à l'instant où une stimulation auriculaire doit être délivrée en l'absence d'événement spontané détecté dans l'oreillette. Le document US-A. 2001/0029392 décrit un appareil médical actif implantable comme décrit dans le préambule de la revendication 1 de ladite demande.

On constate ainsi que, dans un certain nombre de situations qui seront décrites plus en détail par la suite, les appareils connus présentent des limites dans leur capacité à détecter ou à gérer correctement l'activité électrique cardiaque en cas de survenue d'ESV à certains instants critiques de l'onde cardiaque.

Cette incapacité à gérer correctement toutes les situations susceptibles de se présenter en cas de survenue d'ESV a pour effet :
- soit de délivrer au coeur des stimulations auriculaires ou ventriculaires inappropriées, donc inefficaces - voire dangereuses - sur le plan hémodynamique,
- soit, avec les dispositifs AAI/DDD à commutation automatique de mode, de provoquer des basculements intempestifs vers le mode DDD du fait d'un faux diagnostic de bloc auriculo-ventriculaire (le dispositif croit détecter l'apparition d'un BAV, alors qu'en réalité la conduction spontanée est présente),
- soit un cumul des deux actions erronées, c'est-à-dire stimulation inappropriée suivie d'un basculement inutile en mode DDD.

Ces actions inappropriées peuvent créer chez certains patients vulnérables des conditions propices à l'apparition de troubles du rythme ventriculaire, qu'il est important de pouvoir prévenir.

À cet effet, le dispositif de l'invention propose un dispositif médical implantable actif tel que stimulateur cardiaque, défibrillateur et/ou cardioverteur, comprenant :
- des moyens de détection d'événements auriculaires et ventriculaires spontanés, incluant des moyens aptes à détecter la survenue d'extrasystoles ventriculaires parmi les événements ventriculaires spontanés,
- des moyens de stimulation auriculaire, incluant des moyens aptes à calculer un intervalle d'échappement auriculaire et déclencher le comptage de cet intervalle d'échappement auriculaire sur détection d'un événement auriculaire, et à délivrer une stimulation auriculaire à la fin de l'intervalle d'échappement auriculaire si aucun événement auriculaire spontané n'a été détecté dans l'intervalle,

Selon l'invention, ce dispositif comprend en outre :
- des moyens aptes à définir une période critique précédant ou suivant la fin de l'intervalle d'échappement auriculaire telle qu'évaluée par les moyens de stimulation auriculaire,
- des moyens aptes à déterminer, en cas de détection d'une extrasystole ventriculaire, si l'instant de survenue de cette extrasystole ventriculaire se situe dans ladite période critique, de manière à reconnaître ainsi l'apparition d'une condition de risque de fonctionnement inapproprié et/ou de faux diagnostic du dispositif liée à la proximité temporelle de l'extrasystole ventriculaire et de la fin de l'intervalle d'échappement auriculaire, et
- des moyens aptes, dans l'affirmative, à modifier au moins un paramètre de fonctionnement du dispositif de manière à éliminer ladite condition de risque et/ou de faux diagnostic.

Ladite période critique peut être :
- une période définie par une fenêtre temporelle précédant la fin de l'intervalle d'échappement auriculaire d'une durée prédéterminée, par exemple précédant de moins de 63 ms la fin de l'intervalle d'échappement auriculaire ;
- une période réfractaire auriculaire post-ventriculaire suivant la fin de l'intervalle d'échappement auriculaire et dont le comptage est déclenché sur détection de l'extrasystole ventriculaire ;
- une période de surveillance de cross-talk auriculo-ventriculaire suivant la fin de l'intervalle d'échappement auriculaire et dont le comptage est déclenché sur délivrance d'une impulsion de stimulation auriculaire à la fin de l'intervalle d'échappement auriculaire ;
- une période de blanking post-stimulation auriculaire suivant la fin de l'intervalle d'échappement auriculaire et dont le comptage est déclenché sur délivrance d'une impulsion de stimulation auriculaire à la fin de l'intervalle d'échappement auriculaire.

Pour les premier et deuxième cas de période critique, les moyens aptes à modifier au moins un paramètre de fonctionnement du dispositif peuvent notamment réinitialiser le comptage de l'intervalle d'échappement auriculaire sur détection de l'extrasystole ventriculaire (ESV).

L'invention s'applique très avantageusement aux dispositifs de type "DDD-CAM" précités, c'est-à-dire comprenant : des moyens de stimulation ventriculaire ; des moyens de diagnostic de bloc atrio-ventriculaire, aptes à détecter l'apparition et la répétition d'événements auriculaires, spontanés ou stimulés, non suivis de la détection d'un événement ventriculaire spontané associé ; des moyens de commutation de mode, aptes à commander le basculement d'un mode AAI en un mode DDD en cas de diagnostic avéré d'un bloc atrio-ventriculaire ; dispositif caractérisé en ce que lesdits moyens aptes à modifier au moins un paramètre de fonctionnement du dispositif sont des moyens aptes à inhiber lesdits moyens de commutation de mode.

Pour les deuxième et quatrième cas de période critique, les moyens aptes à modifier au moins un paramètre de fonctionnement du dispositif peuvent alors notamment : en cas de survenue d'une extrasystole ventriculaire, mesurer le laps de temps séparant le dernier événement auriculaire détecté et l'instant de survenue de cette extrasystole ventriculaire ; positionner un indicateur de suspicion de faux diagnostic si (i) le laps de temps ainsi mesuré se situe à l'intérieur d'un intervalle de temps prédéterminé, inférieur à la durée de l'intervalle d'échappement auriculaire telle qu'évaluée par les moyens de stimulation auriculaire, et/ou si (ii) une stimulation ventriculaire est délivrée à l'intérieur d'une fenêtre de sécurité suivant la délivrance d'une stimulation auriculaire ; détecter et compter les stimulations auriculaires non suivies d'un événement ventriculaire spontané associé ; et inhiber la commutation de mode pendant un premier nombre de cycles prédéterminé, par exemple douze cycles, si (i) au moins une stimulation auriculaire non suivie d'un événement ventriculaire spontané associé a été détectée et (ii) l'indicateur de suspicion de faux diagnostic est à l'état positionné.

Ledit intervalle de temps prédéterminé est par exemple un intervalle inférieur de 63 ms à la durée de l'intervalle d'échappement auriculaire.

L'indicateur de suspicion de faux diagnostic peut être rétabli à son état initial si aucune stimulation auriculaire non suivie d'un événement ventriculaire spontané associé n'a été détectée pendant un second nombre de cycles prédéterminé, par exemple douze cycles.

Pour les deuxième, troisième et quatrième cas de période critique, les moyens aptes à modifier au moins un paramètre de fonctionnement du dispositif peuvent notamment : en cas de survenue d'une extrasystole ventriculaire, mesurer le laps de temps séparant le dernier événement auriculaire détecté et l'instant de survenue de cette extrasystole ventriculaire ; et prolonger l'intervalle d'échappement auriculaire d'une durée prédéterminée si (i) le laps de temps ainsi mesuré se situe à l'intérieur d'un intervalle de temps prédéterminé, inférieur à la durée de l'intervalle d'échappement auriculaire telle qu'initialement évaluée par les moyens de stimulation auriculaire, et/ou si (ii) une stimulation ventriculaire est délivrée à l'intérieur d'une fenêtre de sécurité suivant la délivrance d'une stimulation auriculaire.

Ladite durée prédéterminée de prolongation de l'intervalle d'échappement auriculaire est par exemple de 63 ms.

L'intervalle d'échappement auriculaire peut être rétabli à sa valeur initiale si aucune stimulation auriculaire non suivie d'un événement ventriculaire spontané associé n'a été détectée pendant un nombre de cycles prédéterminé, par exemple douze cycles.

On va maintenant décrire un exemple de mise en oeuvre du dispositif de l'invention, en référence aux dessins annexés.

La figure 1a illustre un premier cas où la survenue d'une ESV en une période critique induit un fonctionnement inapproprié, pour un dispositif de l'art antérieur.

La figure 1b illustre la même situation que la figure 1a, en montrant la manière dont l'invention permet d'éviter ce fonctionnement inapproprié.

Les figures 2a et 2b sont homologues des figures 1a et 1b, pour un deuxième cas de survenue d'une ESV en période critique.

La figure 3 illustre un troisième cas de survenue d'une ESV en période critique, pour un dispositif de l'art antérieur.

La figure 4 illustre un quatrième cas de survenue d'une ESV en période critique, pour un dispositif de l'art antérieur.

La figure 5 est un organigramme illustrant un algorithme de diagnostic de BAV de type II, adapté selon l'invention pour suspendre le traitement de ce BAV II en cas de détection d'un faux positif résultant de la survenue d'une ESV dans une période critique.

L'invention peut être mise en oeuvre par une programmation appropriée du logiciel de commande d'un stimulateur connu de type double chambre intégrant un mode DDD et un mode AAI avec surveillance de l'activité ventriculaire.

Toutefois, comme on l'a indiqué plus haut, et comme on le verra par la suite, par certains aspects l'invention peut également s'appliquer à des stimulateurs fonctionnant seulement en mode AAI, par aménagement des conditions de mise en oeuvre de ce mode.

On va tout d'abord donner un certain nombre de définitions utilisées dans la suite de la description.
*Détection P* : recueil d'une activité spontanée ayant son origine dans l'oreillette ; on considérera qu'il y a effectivement détection P si celle-ci n'est pas suivie dans un délai donné, par exemple dans les 31 ms, par une détection ventriculaire (sinon, on se trouverait dans une situation de *"far-field* ventriculaire", c'est-à-dire de recueil via l'oreillette d'une dépolarisation lointaine provenant du ventricule).
*Détection R* : recueil d'une activité spontanée ayant son origine dans le ventricule.
*Stimulation A* : stimulation délivrée à l'oreillette.
*Stimulation V:* stimulation délivrée au ventricule.
*Événement auriculaire* : détection P ou bien stimulation A.
*Événement ventriculaire* : détection R ou bien stimulation V.
*Cycle cardiaque* : intervalle de temps séparant deux événements de même nature dans la même cavité, par exemple séparant deux détection P, ou deux stimulations A.
*PP moyen* : intervalle moyen du rythme auriculaire, calculé par exemple sur huit cycle cardiaques ne comprenant pas d'extrasystole.
*Intervalle d'échappement auriculaire (IEA)* : intervalle de temps, compté après une détection ou une stimulation dans l'oreillette, à l'issue duquel une stimulation est délivrée à cette oreillette si aucun événement spontané n'a été détecté dans cette même cavité.
*Extrasystole ventriculaire (ESV)* : une détection ventriculaire est une ESV lorsqu'elle est précédée d'une détection ou d'une stimulation ventriculaire, et lorsque l'intervalle de couplage (intervalle RR ou VR) est inférieur ou égal à une valeur paramétrable, par exemple 75 % du PP moyen.

Pour de plus amples détails sur la détection et le traitement des extrasystoles, on pourra se référer au EP-A-0 550 342 (Ela Médical).

On va maintenant exposer la manière dont est mise en oeuvre l'invention. Au départ, le fonctionnement du stimulateur est un fonctionnement en mode AAI avec surveillance de l'activité ventriculaire, c'est-à-dire qu'une détection auriculaire hors période réfractaire (détection P) ou une stimulation auriculaire (stimulation A) ne démarrent pas de DAV, mais démarrent un intervalle d'échappement auriculaire IEA.

Le mode AAI étant pourvu d'une surveillance de l'activité ventriculaire, l'algorithme recherche par ailleurs la présence ou l'absence d'une activité ventriculaire, qui dans ce dernier cas pourrait laisser suspecter un BAV, de manière à éventuellement basculer en mode DDD de stimulation double chambre avec association atrio-ventriculaire, c'est-à-dire avec calcul et application d'un DAV.

Trois situations peuvent induire le passage au mode DDD :
1°) le bloc auriculo-ventriculaire complet, qui se manifeste par des ondes auriculaires (stimulées ou spontanées) "bloquées", c'est-à-dire des dépolarisations auriculaires non suivies de dépolarisation ventriculaire. Typiquement, la commutation intervient en cas d'une succession de deux ondes bloquées, détectées ou stimulées, ou s'il s'écoule plus de trois secondes sans détection ventriculaire ;
2°) le bloc auriculo-ventriculaire de second degré (BAV II), qui se manifeste par un rapport : nombre d'ondes P non extrasystoliques/nombre d'ondes R non extrasystoliques, supérieur à 1. La commutation intervient si, typiquement, l'appareil détecte un rapport supérieur ou égal à 12/9 ;
3°) le bloc auriculo-ventriculaire du premier degré (BAV I), qui se manifeste par un délai de conduction auriculo-ventriculaire supérieur à une valeur donnée, typiquement 350 ms après une onde P spontanée ou 450 ms après une onde P stimulée, la commutation en mode DDD intervenant après six cycles consécutifs.

En présence de l'un de ces critères, le dispositif bascule du mode AAI en mode DDD.

Après retour d'une activité ventriculaire spontanée sur un certain nombre de cycles, ou après un nombre prédéterminé de cycles en mode DDD, le dispositif commute alors de DDD en AAI et demeure en mode AAI tant qu'aucun des trois critères précités de commutation de AAI vers DDD n'est vérifié.

Les études cliniques ont révélé cependant plusieurs cas dans lesquels le fonctionnement traditionnel en mode AAI et/ou la commutation AAI/DDD n'opère de manière appropriée, du fait de la survenue d'une ESV à une période critique particulière du rythme cardiaque.

Le premier cas, illustré figure 1a, est celui où la détection auriculaire (onde P2 sur la figure 1a) précède de peu, typiquement de moins de 30 ms, la dépolarisation ventriculaire extrasystolique (ESV). Avec un dispositif connu, cette détection auriculaire P2 sera invalidée par l'algorithme de détection et d'analyse du rythme cardiaque, car le dispositif n'est pas capable de discriminer entre :
- une véritable dépolarisation auriculaire spontanée, et
- une situation de *"far-field* ventriculaire" dans l'oreillette, c'est-à-dire une détection auriculaire qui ne résulte pas d'une dépolarisation de l'oreillette, mais qui est un signal capté dans l'oreillette provenant de la détection de l'ESV "entendue dans le lointain" avec retard.

Dans le doute, le dispositif considère qu'il n'y a pas eu d'onde P et donc, conformément au mode de fonctionnement AAI, ne recycle pas l'IEA (le mode AAI pur étant un mode sans détection de l'activité ventriculaire). A la fin de l'intervalle d'échappement IEA, comme aucune détection auriculaire spontanée n'a été reconnue et validée depuis l'onde P1, le stimulateur délivre une stimulation auriculaire à la fin de l'intervalle d'échappement IEA, induisant une dépolarisation auriculaire A1.

Par rapport à la précédente dépolarisation auriculaire spontanée P2 qui a été invalidée par l'algorithme d'analyse du rythme, cette dépolarisation auriculaire stimulée A1 sera prématurée, avec le risque d'induire un trouble du rythme auriculaire si elle survient dans la période de vulnérabilité de l'oreillette.

Enfin, même si cette stimulation induit une dépolarisation auriculaire efficace, c'est-à-dire induisant elle-même une dépolarisation ventriculaire (R2 sur la figure 1a), cette dernière sera prématurée par rapport à l'événement ventriculaire précédent, c'est-à-dire l'événement extrasystolique ESV. De telles conditions sont connues pour générer des troubles du rythme ventriculaire.

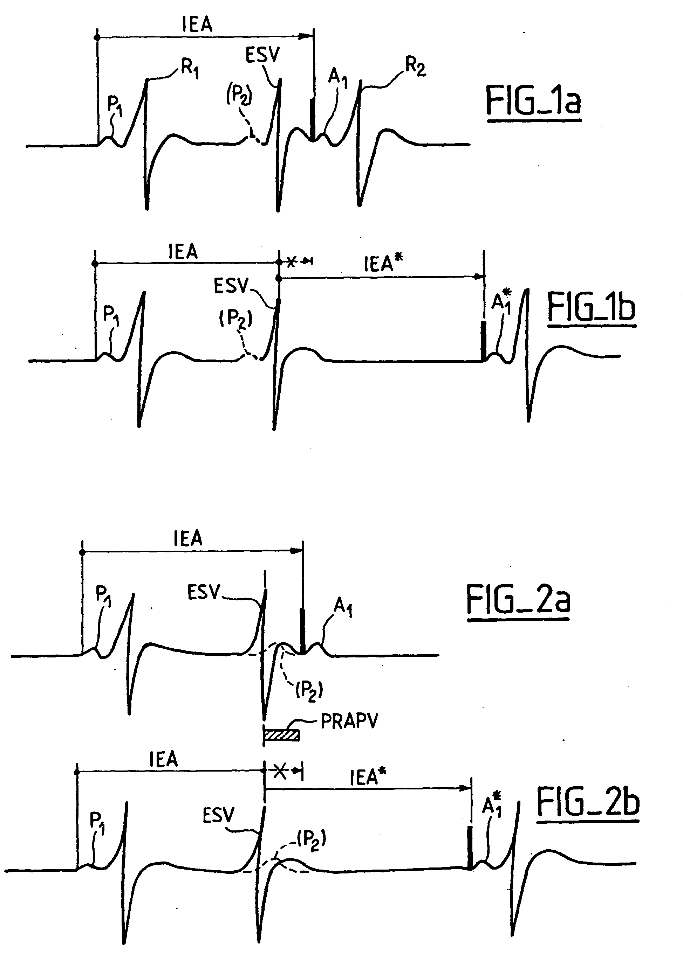

La solution proposée par l'invention, illustrée figure 1b, consiste à recycler l'IEA sur détection d'une ESV. Le recyclage de l'IEA sur cet événement ventriculaire aura le même effet que s'il avait été généré par un événement auriculaire spontané. Le nouvel intervalle IEA* aura pour effet de retarder dans le temps la délivrance de l'impulsion de stimulation auriculaire en l'absence de dépolarisation spontanée (onde A1* sur la figure 1b).

Par rapport à l'onde P2, l'erreur maximale introduite en recyclant l'IEA sur l'événement ventriculaire ESV correspond au cas où l'événement spontané auriculaire survient en fin de période réfractaire post-ESV, ce qui physiologiquement est tout à fait acceptable (c'est-à-dire que le retard maximal de stimulation est égal à la durée de cette période réfractaire).

Inversement, si l'ESV est fortement prématurée, le recyclage sur cet événement ventriculaire sera sans conséquence, car la dépolarisation auriculaire ultérieure qui surviendra après la fin de la période réfractaire post-ESV recyclera de nouveau l'IEA, correspondant au comportement AAI attendu.

Le deuxième cas, illustré figure 2a est celui où la dépolarisation auriculaire P2 intervient non plus peu avant, mais peu après la détection de l'ESV.

Dans ce cas, la dépolarisation auriculaire n'est pas détectée par l'appareil, car elle survient pendant la période réfractaire auriculaire post-ventriculaire (PRAPV).

Cette dépolarisation auriculaire P2 n'étant pas prise en compte, l'IEA n'est pas recyclé et une stimulation auriculaire est donc délivrée en fin d'IEA, induisant une dépolarisation auriculaire indésirable A1.

Cette stimulation auriculaire A1 étant plus prématurée que la dépolarisation auriculaire cachée P2, elle peut être inefficace, c'est-à-dire ne pas induire de dépolarisation ventriculaire associée par les voies naturelles de la conduction. Le stimulateur détecte donc une configuration avec stimulation auriculaire non suivie de dépolarisation ventriculaire, c'est-à-dire une situation de BAV. Si cette séquence se reproduit plus de trois fois sur douze, le dispositif bascule automatiquement en mode DDD, de façon parfaitement inappropriée.

Comme on le voit, ce cas est susceptible de présenter deux inconvénients, à la fois hémodynamique (comme dans le premier cas) du fait d'une stimulation auriculaire intervenant à un instant inapproprié, mais également une erreur de diagnostic de BAV entraînant une commutation intempestive de mode si ce diagnostic se répète.

La solution proposée par l'invention consiste, comme dans le premier cas, à recycler l'IEA sur détection de l'ESV, comme illustré sur la figure 2b : l'IEA est recyclé en IEA*, provoquant une stimulation induisant une dépolarisation auriculaire A1* respectant le rythme naturel du patient (donc satisfaisante sur le plan hémodynamique) et permettant d'induire à son tour une dépolarisation ventriculaire (donc sans détection de BAV) avec une erreur temporelle sur l'instant de délivrance de cette stimulation qui reste dans des limites parfaitement acceptables sur le plan physiologique.

Le recyclage de l'IEA sur l'ESV aura une seconde conséquence, à savoir que la stimulation auriculaire (A1*) ne pourra plus être délivrée prématurément après l'ESV et dépolarisera le ventricule par les voies naturelles de conduction auriculo-ventriculaires, qui sont de nouveau excitables.

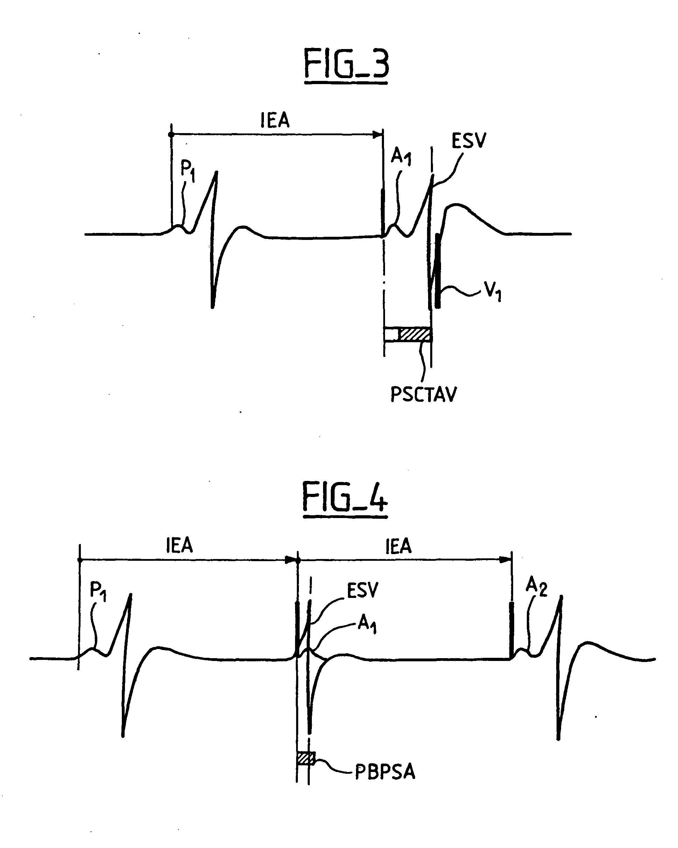

Le troisième cas, illustré sur la figure 3, est celui où, en l'absence de dépolarisation auriculaire spontanée, une stimulation intervient à la fin de l'intervalle d'échappement IEA avec application d'une ESV concomitante à la stimulation de sécurité V1 (voir *infra).*

La stimulation auriculaire A1 en fin d'IEA va déclencher le comptage d'une période dite de surveillance de *cross-talk* auriculo-ventriculaire (PSCTAV), qui est une fenêtre définie typiquement par un intervalle [50 ms, 94 ms] suivant la stimulation auriculaire A1.

Or le dispositif est incapable de différencier entre une véritable dépolarisation ventriculaire (dans le cas présent l'ESV), et la détection dans le ventricule d'un signal qui proviendrait en fait du stimulus électrique auriculaire. Du fait de cette incertitude, par précaution, le dispositif délivre une stimulation ventriculaire de sécurité V1, typiquement 100 ms après la stimulation auriculaire A1. Ce cas n'induit pas de diagnostic de BAV erroné ; cependant il ne s'agit pas d'un comportement AAI correct, car un fonctionnement en mode AAI ne devrait normalement en aucune façon être influencé par l'activité ventriculaire - ce qui n'est pas le cas ici, avec donc les inconvénients corrélatifs sur le plan hémodynamique.

Le quatrième cas, illustré figure 4, est celui où, comme dans le cas précédent, la fin de l'IEA survient quelques millisecondes avant l'ESV, mais l'ESV survenant ici dans la période de *blanking* post-stimulation auriculaire (PBPSA).

A la différence du cas précédent où l'ESV était détectée mais inopérante, ici l'ESV n'est même pas détectée (le *blanking* consiste à bloquer temporairement toute détection du signal après une stimulation, de manière à éviter de saturer les étages amplificateurs).

L'ESV n'étant pas détectée par l'appareil, la stimulation auriculaire A1 déclenchée en fin d'IEA ne pourra pas induire de dépolarisation ventriculaire conduite, car l'ESV rend momentanément réfractaires les tissus de conduction.

Dès lors, le dispositif considère que l'onde A1 est une onde auriculaire bloquée et recycle un nouvel intervalle IEA en indiquant un diagnostic de BAV ; ce diagnostic est en fait un faux positif, car l'absence de dépolarisation ventriculaire conduite tient seulement au fait qu'une ESV est survenue entre-temps, et non à l'apparition d'un défaut de conduction constitutif d'un BAV.

Comme dans le deuxième cas précité, si cette situation se répète plus de trois fois sur douze, le dispositif bascule en mode DDD, de façon inappropriée puisque cette commutation n'est la conséquence d'aucun BAV réel.

Outre le recyclage de l'IEA sur détection de l'ESV, qui permet de résoudre les problèmes liés au premier cas et au deuxième cas précités, l'invention propose deux techniques applicables pour résoudre les problèmes que l'on vient d'exposer liés au troisième cas et au quatrième cas (et accessoirement au deuxième cas précité, aussi).

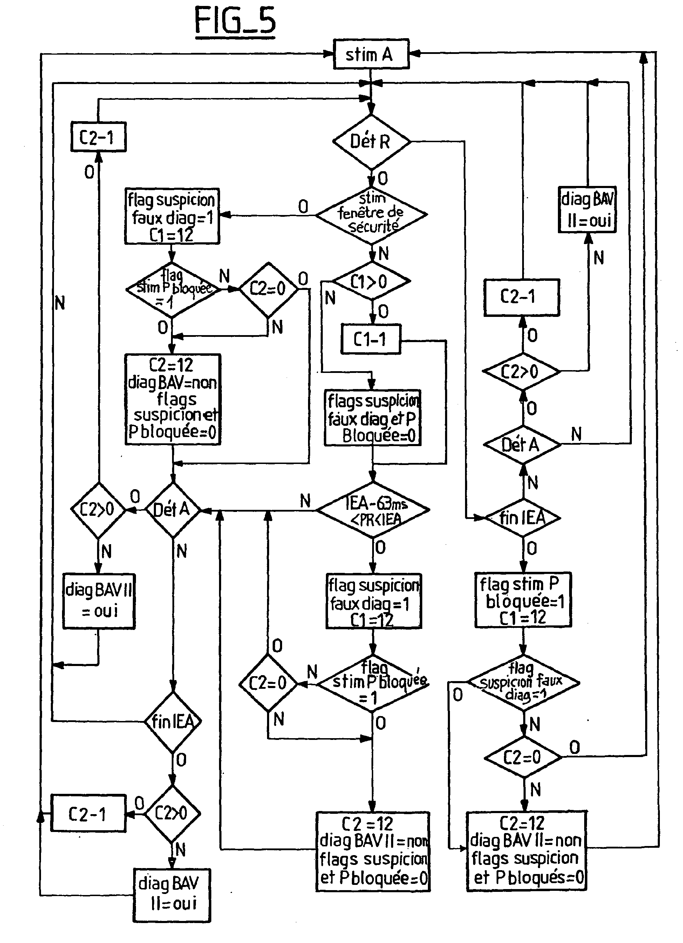

La *première technique,* illustrée par l'organigramme de la figure 5, consiste, lors d'un probable diagnostic de BAV II, à analyser les circonstances ayant conduit à ce diagnostic de BAV de manière à déterminer s'il s'agit d'un véritable BAV II ou d'un faux diagnostic dû à une configuration correspondant à l'un des cas précités et, dans l'affirmative, à suspendre le traitement du BAV II par inhibition de tout passage indésirable en mode DDD.

Outre les abréviations indiquées plus haut *(StimA, DetR,* etc.), on désignera C1 et C2 deux compteurs particuliers mis en oeuvre par cet organigramme.

Le compteur C1 est un compteur initialisé par exemple à 12 (correspondant dans ce cas au numérateur du rapport 12/9 entre le nombre d'ondes P non extrasystoliques et le nombre d'ondes R non extrasystoliques utilisé pour le diagnostic de BAV II comme exposé plus haut). L'initialisation de ce compteur est effectuée lors de la détection d'une ESV suspecte, survenant en fin d'IEA et correspondant au cas indiqué plus haut de stimulation en fenêtre de sécurité, ou au cas d'une ESV dont le couplage est tel qu'elle survient en même temps que la fin de l'IEA.

Ce compteur C1 est décrémenté d'une unité à chaque événement ventriculaire associé à un événement auriculaire ou à chaque ESV dont l'intervalle de couplage présente une durée notablement différente de celle de l'IEA.

Lorsque le compteur C1 atteint une valeur nulle, ceci signifie que depuis douze cycles les événements ventriculaires survenus sont soit des dépolarisations associées à un événement auriculaire correspondant, soit des ESV qui ne surviennent pas en fin d'IEA ; on peut ainsi lever la suspicion de BAV sur des ESV survenant en fin d'IEA.

Le compteur C2, quant à lui, est par exemple initialisé à 12 (valeur donnée à tire d'exemple, définie de la même façon que pour le compteur C1), cette initialisation intervenant lorsque l'algorithme détecte soit une stimulation en fenêtre de sécurité, soit une ESV survenant en fin d'IEA, et qu'une stimulation auriculaire bloquée a été analysée au cours des douze derniers cycles.

Ce compteur C2 est décrémenté d'une unité sur tout événement ventriculaire.

Lorsque C2 > 0, le diagnostic de BAV II est inhibé. Lorsque C2 devient égal à 0, alors le diagnostic de BAV II est avéré.

Plus précisément, lors de la détection d'une ESV, le dispositif mesure l'intervalle entre la dernière onde P et la survenue de cette ESV (intervalle [P,ESV]). Si cet intervalle est voisin de la durée de l'IEA, typiquement si l'on a : IEA - 63 ms < [P,ESV] < IEA, alors un indicateur de suspicion de faux diagnostic est positionné à '1' (indicateur repéré *"flag suspicion faux diag"* sur l'organigramme de la figure 5). Lors d'une stimulation en fenêtre de sécurité, cet indicateur de suspicion est également positionné à '1'.

Si le compteur C2 de stimulations auriculaires bloquées est supérieur ou égal à 1, et que l'indicateur de suspicion de faux diagnostic est également à '1', alors le diagnostic de BAV II est inhibé pendant une durée donnée, par exemple typiquement pendant douze cycles. Ainsi, avec trois stimulations auriculaires bloquées sur douze, on ne déclenche pas de basculement en mode DDD - alors qu'avec un dispositif classique on provoquerait une telle commutation.

Si le dispositif ne trouve aucune ESV de durée voisine de l'IEA (au sens indiqué plus haut) ou aucune stimulation en fenêtre de sécurité, et qu'aucune stimulation auriculaire bloquée n'est détectée pendant, par exemple, douze cycles, alors l'indicateur de suspicion de faux diagnostic est remis à zéro, de même que le compteur C2.

Sur toute détection d'un quelconque des trois événements décrits précédemment (c'est-à-dire d'un évènement qui provoque le positionnement à '1' de l'indicateur de suspicion de faux diagnostic), alors le compteur C1 d'inhibition du diagnostic de BAV II est réinitialisé à la valeur typique d'origine, c'est-à-dire à 12 dans l'exemple donné plus haut. Ceci a pour effet de suspendre le basculement en mode DDD, le diagnostic de BAV II n'ayant pas été avéré.

Une *seconde technique* consiste, lors de la détection d'une ESV proche de la fin de l'IEA (au sens indiqué ci-dessus pour la première technique), ou d'une stimulation en fenêtre de sécurité, à prolonger l'intervalle d'échappement auriculaire IEA, par exemple d'une durée de 63 ms. Le prolongement de l'intervalle d'échappement permet d'éviter tout conflit entre une détection correcte de l'ESV et un comportement AAI satisfaisant.

Ce prolongement perdurera pendant un temps donné, par exemple pendant douze cycles, à moins que l'un des deux évènements ci-dessus ne soit à nouveau détecté par le dispositif.

## Revendications

1. Un dispositif médical implantable actif tel que stimulateur cardiaque, défibrillateur et/ou cardioverteur, comprenant :
- des moyens de détection d'événements auriculaires et ventriculaires spontanés, incluant des moyens aptes à détecter la survenue d'extrasystoles ventriculaires (ESV) parmi les événements ventriculaires spontanés,
- des moyens de stimulation auriculaire, incluant des moyens aptes à calculer un intervalle d'échappement auriculaire (IEA) et déclencher le comptage de cet intervalle d'échappement auriculaire sur détection d'un événement auriculaire, et à délivrer une stimulation auriculaire (A1) à la fin de l'intervalle d'échappement auriculaire si aucun événement auriculaire spontané n'a été détecté dans l'intervalle,
dispositif **caractérisé en ce qu'**il comprend en outre :
- des moyens aptes à définir une période critique précédant ou suivant la fin de l'intervalle d'échappement auriculaire telle qu'évaluée par les moyens de stimulation auriculaire,
- des moyens aptes à déterminer, en cas de détection d'une extrasystole ventriculaire, si l'instant de survenue de cette extrasystole ventriculaire se situe dans ladite période critique, de manière à reconnaître ainsi l'apparition d'une condition de risque de fonctionnement inapproprié et/ou de faux diagnostic du dispositif liée à la proximité temporelle de l'extrasystole ventriculaire et de la fin de l'intervalle d'échappement auriculaire, et
- des moyens aptes, dans l'affirmative, à modifier au moins un paramètre de fonctionnement du dispositif de manière à éliminer ladite condition de risque et/ou de faux diagnostic.

2. Le dispositif de la revendication 1, dans lequel ladite période critique est une période définie par une fenêtre temporelle précédant la fin de l'intervalle d'échappement auriculaire (IEA) d'une durée prédéterminée.

3. Le dispositif de la revendication 1, dans lequel ladite période critique est une période définie par une fenêtre temporelle précédant de moins de 63 ms la fin de l'intervalle d'échappement auriculaire.

4. Le dispositif de la revendication 1, dans lequel ladite période critique est une période réfractaire auriculaire post-ventriculaire (PRAPV) suivant la fin de l'intervalle d'échappement auriculaire (IEA) et dont le comptage est déclenché sur détection de l'extrasystole ventriculaire (ESV).

5. Le dispositif de la revendication 1, dans lequel ladite période critique est une période de surveillance de cross-talk auriculo-ventriculaire (PSC-TAV) suivant la fin de l'intervalle d'échappement auriculaire (IEA) et dont le comptage est déclenché sur délivrance d'une impulsion de stimulation auriculaire (A1) à la fin de l'intervalle d'échappement auriculaire (IEA).

6. Le dispositif de la revendication 1, dans lequel ladite période critique est une période de blanking post-stimulation auriculaire (PBPSA) suivant la fin de l'intervalle d'échappement auriculaire (IEA) et dont le comptage est déclenché sur délivrance d'une impulsion de stimulation auriculaire (A1) à la fin de l'intervalle d'échappement auriculaire (IEA).

7. Le dispositif de l'une des revendications 2 ou 4, dans lequel lesdits moyens aptes à modifier au moins un paramètre de fonctionnement du dispositif sont des moyens aptes à réinitialiser le comptage de l'intervalle d'échappement auriculaire (IEA*) sur détection de l'extrasystole ventriculaire (ESV).

8. Le dispositif de la revendication 1, du type comprenant en outre :
- des moyens de stimulation ventriculaire,
- des moyens de diagnostic de bloc atrio-ventriculaire, aptes à détecter l'apparition et la répétition d'événements auriculaires, spontanés ou stimulés, non suivis de la détection d'un événement ventriculaire spontané associé,
- des moyens de commutation de mode, aptes à commander le basculement d'un mode AAI en un mode DDD en cas de diagnostic avéré d'un bloc atrio-ventriculaire,
dispositif **caractérisé en ce que** lesdits moyens aptes à modifier au moins un paramètre de fonctionnement du dispositif sont des moyens aptes à inhiber lesdits moyens de commutation de mode.

9. Le dispositif de l'une des revendications 4 ou 6, dans lequel lesdits moyens aptes à modifier au moins un paramètre de fonctionnement du dispositif sont des moyens aptes à :
- en cas de survenue d'une extrasystole ventriculaire (ESV), mesurer le laps de temps séparant le dernier événement auriculaire détecté (P1) et l'instant de survenue de cette extrasystole ventriculaire (ESV),
- positionner un indicateur de suspicion de faux diagnostic si (i) le laps de temps ainsi mesuré se situe à l'intérieur d'un intervalle de temps prédéterminé, inférieur à la durée de l'intervalle d'échappement auriculaire telle qu'évaluée par les moyens de stimulation auriculaire, et/ou si (ii) une stimulation ventriculaire est délivrée à l'intérieur d'une fenêtre de sécurité suivant la délivrance d'une stimulation auriculaire,
- détecter et compter les stimulations auriculaires non suivies d'un événement ventriculaire spontané associé, et
- inhiber la commutation de mode pendant un premier nombre de cycles prédéterminé si (i) au moins une stimulation auriculaire non suivie d'un événement ventriculaire spontané associé a été détectée et (ii) l'indicateur de suspicion de faux diagnostic est à l'état positionné.

10. Le dispositif de la revendication 9, dans lequel ledit intervalle de temps prédéterminé est un intervalle inférieur de 63 ms à la durée de l'intervalle d'échappement auriculaire.

11. Le dispositif de la revendication 9, dans lequel ledit premier nombre de cycles prédéterminé est de douze cycles.

12. Le dispositif de la revendication 9, dans lequel lesdits moyens aptes à modifier au moins un paramètre de fonctionnement du dispositif sont également aptes à :
- rétablir l'indicateur de suspicion de faux diagnostic à son état initial si aucune stimulation auriculaire non suivie d'un événement ventriculaire spontané associé n'a été détectée pendant un second nombre de cycles prédéterminé.

13. Le dispositif de la revendication 12, dans lequel ledit second nombre de cycles prédéterminé est de douze cycles.

14. Le dispositif de l'une des revendications 4, 5 ou 6, dans lequel lesdits moyens aptes à modifier au moins un paramètre de fonctionnement du dispositif sont des moyens aptes à :
- en cas de survenue d'une extrasystole ventriculaire (ESV), mesurer le laps de temps séparant le dernier événement auriculaire détecté (P1) et l'instant de survenue de cette extrasystole ventriculaire (ESV), et
- prolonger l'intervalle d'échappement auriculaire d'une durée prédéterminée si (i) le laps de temps ainsi mesuré se situe à l'intérieur d'un intervalle de temps prédéterminé, inférieur à la durée de l'intervalle d'échappement auriculaire telle qu'initialement évaluée par les moyens de stimulation auriculaire, et/ou si (ii) une stimulation ventriculaire est délivrée à l'intérieur d'une fenêtre de sécurité suivant la délivrance d'une stimulation auriculaire.

15. Le dispositif de la revendication 9, dans lequel ladite durée prédéterminée de prolongation de l'intervalle d'échappement auriculaire est de 63 ms.

16. Le dispositif de la revendication 14, dans lequel lesdits moyens aptes à modifier au moins un paramètre de fonctionnement du dispositif sont également aptes à :
- rétablir l'intervalle d'échappement auriculaire à sa valeur initiale si aucune stimulation auriculaire non suivie d'un événement ventriculaire spontané associé n'a été détectée pendant un nombre de cycles prédéterminé.

17. Le dispositif de la revendication 16, dans lequel ledit nombre de cycles prédéterminé est de douze cycles.

## Claims

1. An active implantable medical device, such as a pacemaker, defibrillator and/or cardioverter, comprising:
- means for detecting spontaneous atrial and ventricular events, including means able to detect the occurrence of ventricular extra-systoles (VES) among the spontaneous ventricular events,
- atrial stimulation means, including means able to calculate an atrial escape interval (AEI) and to trigger the counting of this atrial escape interval upon detection of an atrial event, and to deliver an atrial stimulation (A1) at the end of the atrial escape interval if no spontaneous atrial event has been detected in the interval,
a device, **characterised in that** it further comprises:
- means able to define a critical period preceding or following the end of the atrial escape interval as evaluated by the atrial stimulation means,
- means able to determine, in case of the detection of a ventricular extrasystole, whether the moment of occurrence of this ventricular extrasystole is situated within the critical period, in order to identify the appearance of a condition with a risk of an inappropriate functioning and/or misdiagnosis of the device linked to the temporal proximity of the ventricular extrasystole and the end of the atrial escape interval, and
- means able to, in the affirmative, modify at least one function parameter of the device in order to eliminate said risk and/or misdiagnosis condition.

2. The device according to claim 1, wherein said critical period is a period defined by a time frame preceding the end of the atrial escape interval (AEI) by a predetermined duration.

3. The device according to claim 1, wherein said critical period is a period defined by a time frame preceding the end of the atrial escape interval by less than 63 ms.

4. The device according to claim 1, wherein said critical period is a refractory atrial post-ventricular period (RAPVP) following the end of the atrial escape interval (AEI), and the counting of which is triggered upon detection of the ventricular extrasystole (VES).

5. The device according to claim 1, wherein said critical period is a period of monitoring atrioventricular cross-talk (PMAVC-T) following the end of the atrial escape interval (AEI), and the counting of which is triggered upon delivery of an atrial stimulation pulse (A1) at the end of the atrial escape interval (AEI).

6. The device according to claim 1, wherein said critical period is a period of atrial post-stimulation blanking (PAPSB) following the end of the atrial escape interval (AEI), and the counting of which is triggered upon delivery of an atrial stimulation pulse (A1) at the end of the atrial escape interval (AEI).

7. The device according to one of claims 2 or 4, wherein said means able to modify at least one function parameter of the device is a means able to reinitialise the counting of the atrial escape interval (AEI*) upon detection of the ventricular extrasystole (VES).

8. The device according to claim 1, of the type further comprising:
- ventricular stimulation means,
- means for diagnosing an atrioventricular block, able to detect the occurrence and the repetition of spontaneous or stimulated atrial events, which are not followed by the detection of an associated spontaneous ventricular event,
- mode commutation means, able to command the shift from an AAI-mode to a DDD-mode in the case of a proven diagnosis of an atrioventricular block,
the device being **characterised in that** said means able to modify at least one function parameter of the device is a means able to inhibit said mode commutation means.

9. The device according to one of claims 4 or 6, wherein said means able to modify at least one function parameter of the device is a means able to:
- in the case of an occurrence of a ventricular extrasystole (VES), measure the time span separating the last detected atrial event (P1) and the moment of occurrence of this ventricular extrasystole (VES),
- position a misdiagnosis suspicion indicator if (i) the time span such measured is located within a predetermined time interval, less than the duration of the atrial escape interval as evaluated by the atrial stimulation means, and/or if (ii) a ventricular stimulation is delivered within a security frame following the delivery of an atrial stimulation,
- detect and count the atrial stimulations that are not followed by an associated spontaneous ventricular event, and
- inhibit the mode commutation during a first predetermined number of cycles if (i) at least one atrial stimulation that is not followed by an associated spontaneous ventricular event has been detected and (ii) the misdiagnosis suspicion indicator is in the positioned state.

10. The device according to claim 9, wherein said predetermined time interval is an interval that is 63 ms less than the duration of the atrial escape interval.

11. The device according to claim 9, wherein said first predetermined number of cycles is twelve cycles.

12. The device according to claim 9, wherein said means able to modify at least one function parameter of the device is also able to:
- return the misdiagnosis suspicion indicator to its initial state if no atrial stimulation not followed by an associated spontaneous ventricular event has been detected during a second predetermined number of cycles.

13. The device according to claim 12, wherein said second predetermined number of cycles is twelve cycles.

14. The device according to one of claims 4, 5 or 6, wherein said means able to modify at least one function parameter of the device is a means able to:
- in the case of the occurrence of a ventricular extrasystole (VES), measure the time span separating the last detected atrial event (P1) and the moment of occurrence of this ventricular extrasystole (VES), and
- prolong the atrial escape interval by a predetermined duration if (i) the time span such measured is located within a predetermined time interval, less than the duration of the atrial escape interval as initially evaluated by the atrial stimulation means, and/or if (ii) a ventricular stimulation is delivered within a security frame following the delivery of an atrial stimulation.

15. The device according to claim 9, wherein said predetermined duration of prolongation of the atrial escape interval is 63 ms.

16. The device according to claim 14, wherein said means able to modify at least one function parameter of the device is also able to:
- re-establish the atrial escape interval to its initial value if no atrial stimulation not followed by an associated spontaneous ventricular event has been detected during a predetermined number of cycles.

17. The device according to claim 16, wherein said predetermined number of cycles is twelve cycles.

## Patentansprüche

1. Aktive implantierbare medizinische Vorrichtung, wie bspw. Herzschrittmacher, Defibrillator und/oder Kardioverter, mit:
- Mitteln zur Erfassung von spontanen Herzvorhof- und Herzkammerereignissen, die Mittel umfassen, welche geeignet sind, das Auftreten von ventrikulären Extrasystolen (VES) unter den spontanen Herzkammerereignissen zu erfassen,
- Mitteln zur Herzvorhofstimulation, welche Mittel umfassen, die geeignet sind, ein atriales Escape-Intervall (AEI) zu berechnen und bei Erfassung eines Herzvorhofereignisses das Auszählen dieses atrialen Escape-Intervalls auszulösen, und eine atriale Stimulation (A1) am Ende des atrialen Escape-Intervalls abzugeben, wenn im Intervall keinerlei spontanes Herzvorhofereignis erfasst wurde,
Vorrichtung, die **dadurch gekennzeichnet ist**, das sie außerdem folgendes umfasst:
- Mitteln, die geeignet sind, eine kritische Periode zu definieren, die dem Ende des atrialen Escape-Intervalls vorausgeht oder nachfolgt, wie es durch die Mittel zur Herzvorhofstimulation bestimmt wird,
- Mitteln, die geeignet sind, im Falle der Erfassung einer ventrikulären Extrasystole festzustellen, ob sich der Zeitpunkt des Auftretens dieser ventrikulären Extrasystole innerhalb der kritischen Periode befindet, um so das Auftauchen eines Zustands mit Risiko einer Fehlfunktion und/oder eines Diagnosefehlers bei der Vorrichtung zu erkennen, der durch die zeitliche Nähe der ventrikulären Extrasystole zum Ende des atrialen Escape-Intervalls bedingt ist, und
- Mitteln, die geeignet sind, im positiven Fall mindestens einen Betriebsparameter der Vorrichtung zu verändern, um so den Risiko-und/oder Fehldlagnosezustand zu unterbinden.

2. Vorrichtung nach Anspruch 1, bei welcher die kritische Periode eine durch ein Zeitfenster definierte Periode ist, das dem Ende des atrialen Escape-Intervalls (AEI) um eine vorbestimmte Dauer vorausgeht.

3. Vorrichtung nach Anspruch 1, bei welcher die kritische Periode eine durch ein Zeitfenster definierte Periode ist, das dem Ende des atrialen Escape-Intervalls um weniger als 63 ms vorausgeht.

4. Vorrichtung nach Anspruch 1, bei welcher die kritische Periode eine refraktäre atriale post-ventrikuläre Periode (RAPVP) ist, die dem Ende des atrialen Escape-Intervalls (AEI) nachfolgt, und deren Auszählen bei Erfassung der ventrikulären Extrasystole (VES) ausgelöst wird.

5. Vorrichtung nach Anspruch 1, bei welcher die kritische Periode eine Periode der Überwachung eines atrial-ventrikulären Übersprechens (PÜAVÜ) ist, welche dem Ende des atrialen Escape-Intervalls (AEI) nachfolgt, und deren Auszählen bei Abgabe eines atrialen Stimulationsimpulses (A1) am Ende des atrialen Escape-Intervalls (AEI) ausgelöst wird.

6. Vorrichtung nach Anspruch 1, bei welcher die kritische Periode eine Periode des Austastens ("blanking") nach atrialer Stimulation (PANAS) ist, die dem Ende des atrialen Escape-Intervalls (AEI) nachfolgt, und deren Auszählen bei Abgabe eines atrialen Stimulationsimpulses (A1) am Ende des atrialen Escape-Intervalls (AEI) ausgelöst wird.

7. Vorrichtung nach einem der Ansprüche 2 oder 4, bei welcher die Mittel, die geeignet sind, mindestens einen Betriebsparameter der Vorrichtung zu verändern, Mittel sind, die geeignet sind, das Auszählen des atrialen Escape-Intervalls (AEI*) bei Erfassung der ventrikulären Extrasystole (VES) zu reinitialisieren.

8. Vorrichtung nach Anspruch 1, vom Typ, der weiterhin folgendes umfasst:
- Mittel zur Herzkammerstimulation,
- Mittel zur Diagnose eines atrioventrikulären Blocks, die geeignet sind, das Auftreten und das sich Wiederholen von spontanen oder stimulierten Herzvorhofereignissen zu erfassen, die nicht von der Erfassung eines zugeordneten spontanen Herzkammereignisses gefolgt werden,
- Mittel zur Modusumschaltung, die geeignet sind, im Falle der erwiesenen Diagnose eines atrioventrikulären Blocks das Umschalten von einem AAI-Modus zu einem DDD-Modus zu befehligen,
Vorrichtung, die **dadurch gekennzeichnet ist, dass** die Mittel, die geeignet sind, mindestens einen Betriebsparameter der Vorrichtung zu verändern, Mittel sind, die geeignet sind, die Mittel zur Modusumschaltung zu unterdrücken.

9. Vorrichtung nach einem der Ansprüche 4 oder 6, bei welcher die Mittel, die geeignet sind, mindestens einen Betriebsparameter der Vorrichtung zu verändern, Mittel sind, die geeignet sind:
- im Falle des Auftretens einer ventrikulären Extrasystole (VES) den Zeitraum zu messen, der das letzte erfasste Herzvorhofereignis (P1) vom Zeitpunkt des Auftretens dieser ventrikulären Extrasystole (VES) trennt,
- eine Anzeige einer Vermutung einer Fehldiagnose zu positionieren, wenn (i) der so gemessene Zeitraum sich innerhalb eines vorbestimmten Zeitintervalls befindet, das geringer als die Dauer des atrialen Escape-Intervalls ist, wie sie durch die Mittel zur atrialen Stimulation bestimmt wird, und/oder wenn (ii) eine ventrikuläre Stimulation innerhalb eines Sicherheitsfensters im Anschluss an die Abgabe einer atrialen Stimulation abgegeben wird,
- die atrialen Stimulationen zu erfassen und zu zählen, die nicht von einem zugeordneten spontanen Herzkammerereignis gefolgt werden, und
- die Modusumschaltung während einer ersten vorbestimmten Zyklenanzahl zu unterdrücken, wenn (i) mindestens eine Herzvorhofstimulation erfasst wurde, die nicht von einem zugeordneten spontanen Herzkammereignis gefolgt wird, und (ii) die Anzeige einer Vermutung einer Fehldiagnose im Positionierten Zustand ist.

10. Vorrichtung nach Anspruch 9, bei welcher das vorbestimmte Zeitintervall ein Intervall ist, das um 63 ms geringer als die Dauer des atrialen Escape-Intervalls ist.

11. Vorrichtung nach Anspruch 9, bei welcher die erste vorbestimmte Zyklenanzahl zwölf Zyklen beträgt.

12. Vorrichtung nach Anspruch 9, bei welcher die Mittel, welche geeignet sind, mindestens einen Betriebsparameter der Vorrichtung zu verändern, ebenfalls geeignet sind:
- die Anzeige einer Vermutung einer Fehldiagnose in ihren Ursprungszustand zurückzusetzen, wenn keinerlei Herzvorhofstimulation, die nicht von einem zugeordneten spontanen Herzkammerereignis gefolgt wird, während einer zweiten vorbestimmten Zyklenanzahl erfasst wurde.

13. Vorrichtung nach Anspruch 12, bei welcher die zweite vorbestimmte Zyklenanzahl zwölf Zyklen beträgt.

14. Vorrichtung nach einem der Ansprüche 4, 5 oder 6, bei welcher die Mittel, welche geeignet sind, mindestens einen Betriebsparameter der Vorrichtung zu verändern, Mittel sind, die geeignet sind:
- im Falle des Auftretens einer ventrikulären Extrasystole (VES) den Zeitraum zu messen, der das letzte erfasste Herzvorhofereignis (P1) vom Zeitpunkt des Auftretens dieser ventrikulären Extrasystole (VES) trennt, und
- das atriale Escape-Intervall um eine vorbestimmte Dauer zu verlängern, wenn (i) der so gemessene Zeitraum sich innerhalb eines vorbestimmten Zeitintervalls befindet, das geringer als die Dauer des atrialen Escape-Intervalls ist, wie sie ursprünglich durch die Mittel zur Herzvorhofstimulation bestimmt wurde, und/oder wenn (ii) eine Herzkammerstimulation innerhalb eines Sicherheitsfensters im Anschluss an die Abgabe einer Herzvorhofstimulation abgegeben wird.

15. Vorrichtung nach Anspruch 9, bei welcher die vorbestimmte Dauer zur Verlängerung des atrialen Escape-Intervalls 63 ms beträgt.

16. Vorrichtung nach Anspruch 14, bei welcher die Mittel, welche geeignet sind, mindestens einen Betriebsparameter der Vorrichtung zu verändern, ebenfalls geeignet sind:
- das atriale Escape-Intervall auf seinen Ursprungswert zurückzusetzen, wenn keinerlei Herzvorhofstimulation, die nicht von einem zugeordneten spontanen Herzkammerereignis gefolgt wird, während einer vorbestimmten Zyklenanzahl erfasst wurde.

17. Vorrichtung nach Anspruch 16, bei welcher die vorbestimmte Zyklenanzahl zwölf Zyklen beträgt.
